# DEMANDE DE BREVET EUROPEEN

(11) **EP 3 861 933 A1**
(43) Date de publication de la demande: **11.08.2021**
(21) Numéro de dépôt: 20305108.1
(22) Date de dépôt: 05.02.2020
(51) Int. Cl.: A61B 5/145, A61B 5/1468, A61B 5/00

(54) **DISPOSITIF DE MESURE D'ANALYTE ET D'ENFONCEMENT DE MICROAIGUILLES DANS LA PEAU**

(71) Demandeur: PKvitality, 75001 Paris (FR)
(72) Inventeur: PIERART, Luc, 94800 Villejuif (FR); JARRI, Khaled, 66100 Perpignan (FR)
(74) Mandataire: Regimbeau

(57) **Abrégé**

La présente invention concerne un dispositif de mesure d'analyte présent dans un liquide interstitiel, le dispositif dans lequel l'unité de contrôle est configurée pour imposer un courant électrique et/ou une tension électrique entre une première électrode de conductivité et une deuxième électrode de conductivité, et mesurer une valeur représentative de la conductivité entre la première électrode de conductivité et la deuxième électrode de conductivité, et enregistrer une donnée représentative d'un enfoncement dans la peau d'une ou de premières microaiguilles de mesure d'analyte suffisant pour une mise en œuvre d'une mesure d'analyte lorsque la valeur mesurée est représentative d'une conductivité supérieure à une première conductivité seuil prédéterminée *σₜ₁*.

## Description

### DOMAINE DE L'INVENTION

La présente invention concerne un dispositif de surveillance corporelle via analyse de liquide corporel, typiquement interstitiel, à l'aide de microaiguilles.

Plus précisément, la présente invention concerne la gestion de l'enfoncement des microaiguilles dans la peau permettant de mesurer la présence d'un analyte dans le liquide corporel.

### ETAT DE LA TECHNIQUE

Certaines pathologies comme le diabète nécessitent une surveillance quotidienne de paramètres biochimiques du corps humain, *i.e.* des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

On connait des dispositifs avec microaiguilles, qui ont l'avantage d'être moins invasive que des aiguilles classiques. Toutefois, il est important que ces microaiguilles restent en place.

Il existe pour cela des dispositifs à demeure, où des microaiguilles sont maintenues sur la peau avec une bande adhésive. Toutefois, on souhaite pouvoir effectuer un contrôle en continu ou quasi-continu, ce qui requiert des dispositifs autonomes. On pourra citer le dispositif GlucoWatch, qui utilisait l'iontophorèse (et non pas des aiguilles).

Toutefois, lorsqu'un tel dispositif comporte des microaiguilles, il peut exister des interférences liées à la sueur présente sur la peau. Ces interférences dépendent en outre de l'épaisseur de l'épiderme, qui est variable selon les individus : l'épaisseur de peau pour atteindre le liquide interstitiel n'est alors pas la même.

Pour résoudre certains des problèmes précités, il existe des solutions visant à masquer certaines parties des microaiguilles, pour éviter qu'elles n'interfèrent. On peut citer un revêtement isolant, comme dans le document US 2014/0275897 par exemple. Toutefois, ces produits multicouches sont coûteux à réaliser et difficile à produire.

De plus, de par la taille des microaiguilles, il existe un risque que ces microaiguilles soient mal enfoncées dans la peau : la mesure en résultant est alors faussée. Dans le cas d'une mesure par conductimétrie, il est par exemple possible de mesurer la conductance de la sueur à la surface de la peau au lieu de celle du liquide corporel.

### EXPOSE DE L'INVENTION

Un but de l'invention est de proposer une solution pour contrôler l'enfoncement dans la peau de microaiguilles de mesure d'un analyte dans un fluide corporel.

Ce but est atteint dans le cadre de la présente invention grâce à un dispositif de mesure d'analyte présent dans un liquide interstitiel, le dispositif comprenant :
- un boîtier comprenant un substrat,
- une unité de contrôle, le boîtier comprenant l'unité de contrôle,
- au moins une première microaiguille configurée pour être insérée dans la peau d'un utilisateur,
   la première microaiguille comprenant une base montée fixe sur le substrat et une tête ponctuelle de la première microaiguille, la base et la tête de la première microaiguille étant distantes d'une première hauteur *h₁*, la première microaiguille présentant une face latérale comprise entre la base exclue et la tête, la face latérale comprenant une face métallique s'étendant continûment depuis la tête,

- une pluralité de microaiguilles de mesure d'analyte configurées pour être insérées dans la peau,
- une première électrode de conductivité et une deuxième électrode de conductivité, la première électrode de conductivité comprenant la face métallique de la ou de chacune des première(s) microaiguille(s), et reliant électriquement la face métallique de la ou de chacune des première(s) microaiguille(s) à l'unité de contrôle,
dans lequel l'unité de contrôle est configurée pour :
a) imposer un courant électrique et/ou une tension électrique entre la première électrode de conductivité et la deuxième électrode de conductivité lorsque le substrat du dispositif est mis en contact avec la peau de l'utilisateur, et mesurer une valeur représentative de la conductivité entre la première électrode de conductivité et la deuxième électrode de conductivité,
b) enregistrer une donnée représentative d'un enfoncement dans la peau de la ou des microaiguilles de mesure d'analyte suffisant pour une mise en œuvre d'une mesure d'analyte lorsque la valeur mesurée lors de l'étape a) est représentative d'une conductivité supérieure à une première conductivité seuil prédéterminée *σₜ₁*.

Le système peut avantageusement comprendre les caractéristiques suivantes, prises individuellement ou en l'une quelconque de leurs combinaisons techniquement possibles :
- la surface de la face métallique, ou la somme des surfaces des faces métalliques, est supérieure ou égale à 0,35 mm², préférentiellement supérieure à 0,5 mm² et notamment supérieure à 1 mm²,
- le dispositif comprend une deuxième microaiguille, la deuxième microaiguille comprenant une base montée fixe sur le substrat et une tête ponctuelle de la deuxième microaiguille, la base et la tête de la deuxième microaiguille étant distantes d'une deuxième hauteur *h₂*, la deuxième microaiguille présentant une face latérale comprise entre la base de la deuxième microaiguille exclue et la tête de la deuxième microaiguille, la face latérale de la deuxième microaiguille comprenant une face métallique s'étendant continûment depuis la tête, la deuxième électrode de conductivité comprenant la face métallique de la deuxième microaiguille et reliant électriquement la face métallique de la deuxième microaiguille à l'unité de contrôle,
- la première hauteur *h₁* et la deuxième hauteur *h₂* sont strictement différentes,
- la première hauteur *h₁* et la deuxième hauteur *h₂* sont égales, et le substrat présente un enfoncement sur lequel est monté fixe la base de la première microaiguille ou la base de la deuxième microaiguille,
- le dispositif comprend une pluralité de premières microaiguilles, les hauteurs *h₁* d'au moins deux premières microaiguilles étant strictement différentes entre-elles et/ou les hauteurs *h₁* des premières microaiguilles étant égales entre-elles, le substrat présentant un enfoncement sur lequel est monté fixe la base d'au moins une des premières microaiguilles,
- la face métallique de la première microaiguille s'étend depuis la tête de la première microaiguille jusqu'à une partie de la face latérale distante de plus de *h*/10, préférentiellement de plus de h/5, et notamment de plus de h/3 du substrat,
- la face métallique de la première microaiguille s'étend depuis la tête de chaque microaiguille jusqu'à une partie de la face latérale distante de moins de 9.*h*/10, préférentiellement de moins de 4.h/5, et notamment de moins de 2.h/3 du substrat,
- la face métallique de la première microaiguille et la face métallique de la deuxième microaiguille s'étendent depuis chacune des têtes de chaque microaiguille jusqu'à des hauteurs strictement différentes,
- le dispositif comprend une troisième électrode de conductivité, et préférentiellement une quatrième électrode de conductivité, le substrat comprenant une première surface de conductivité métallique, et préférentiellement une deuxième surface de conductivité métallique, la troisième électrode de conductivité reliant électriquement l'unité de contrôle à la première surface de conductivité métallique, et préférentiellement la quatrième électrode de conductivité reliant électriquement l'unité de contrôle à la deuxième surface de conductivité métallique,
- la valeur de la première conductivité seuil *σₜ₁* est supérieure ou égale à 0,05 S/m, notamment supérieure à 0,1 S/m et préférentiellement supérieure à 0,3 S/m,
- l'unité de contrôle est configurée pour enregistrer une donnée représentative d'une humidité intense sur la peau de l'utilisateur lorsque la valeur mesurée lors de l'étape a) est représentative d'une conductivité supérieure à une deuxième conductivité seuil *σₜ₂*, la valeur de la deuxième conductivité seuil *σₜ₂* étant préférentiellement supérieure à 0,7 S/m,
- l'unité de contrôle est configurée pour enregistrer une donnée représentative d'un enfoncement d'une première microaiguille insuffisant de la peau de l'utilisateur lorsque la valeur mesurée lors de l'étape a) est représentative d'une conductivité inférieure à la première conductivité seuil *σₜ₁* prédéterminée,
- l'unité de contrôle est configurée pour :
   mesurer la concentration Co d'un analyte présent dans un liquide interstitiel, enregistrer une donnée représentative une donnée représentative d'une humidité modérée sur la peau de l'utilisateur et d'un enfoncement d'une première microaiguille insuffisant de la peau de l'utilisateur lorsque la valeur de la conductivité mesurée lors de l'étape a) est supérieure à la valeur de la première conductivité électrique seuil *σₜ₁*, et lorsque la concentration de l'analyte de mesure est inférieure à une première concentration C₁ prédéterminée ou supérieure à une deuxième concentration C₂ prédéterminée, la première concentration étant préférentiellement inférieure à 2 mM inclus, et notamment à 0,5 mM inclus, la deuxième concentration étant préférentiellement supérieure à 20 mM inclus.

Un autre aspect de l'invention est un procédé détection de l'enfoncement d'une microaiguille de mesure d'analyte d'un dispositif conforme à l'invention au travers de la peau, comprenant au moins :
a) une imposition d'un courant électrique et/ou d'une tension électrique entre la première électrode de conductivité et la deuxième électrode de conductivité lorsque le substrat du dispositif est mis en contact avec la peau de l'utilisateur et de mesure d'une valeur représentative de la conductivité entre la première électrode de conductivité et la deuxième électrode de conductivité,
b) l'enregistrement d'une donnée représentative d'un enfoncement dans la peau de la ou des microaiguilles de mesure d'analyte suffisant pour une mise en œuvre d'une mesure d'analyte lorsque la valeur mesurée lors de l'étape a) est représentative d'une conductivité supérieure à une première conductivité seuil prédéterminée *σₜ₁*.

Avantageusement, lors de l'étape a), l'imposition d'un courant électrique et/ou d'une tension électrique entre la première électrode de conductivité et la deuxième électrode de conductivité est mise en œuvre pendant moins de 300 ms, notamment pendant moins de 200 ms, et préférentiellement pendant moins de 100 ms.

Avantageusement, le procédé comprend :
c) une imposition d'un courant électrique et/ou d'une tension électrique entre la première électrode de conductivité et la deuxième électrode de conductivité lorsque la tête de la ou des premières microaiguille(s) est mise en contact avec la peau de l'utilisateur et une première mesure d'une valeur représentative de la conductivité entre la première électrode de conductivité et la deuxième électrode de conductivité,
d) une imposition d'un courant électrique et/ou d'une tension électrique entre la première électrode de conductivité et la deuxième électrode de conductivité de conductivité lorsque le substrat du dispositif est mis en contact avec la peau de l'utilisateur de manière à enfoncer la ou les premières microaiguilles dans la peau, et mesure d'une valeur représentative de la conductivité entre la première électrode de conductivité et la deuxième électrode de conductivité, e) une étape de détermination de la première conductivité seuil *σₜ₁* à partir de la valeur mesurée lors de l'étape c) et à partir de la valeur mesurée lors de l'étape d).

Avantageusement, le dispositif comprend en outre une troisième électrode de conductivité et une quatrième électrode de conductivité, le substrat comprenant une première surface de conductivité métallique, et une deuxième surface de conductivité métallique, la troisième électrode de conductivité reliant électriquement l'unité de contrôle à la première surface de conductivité métallique, et la quatrième électrode de conductivité reliant électriquement l'unité de contrôle à la deuxième surface de conductivité métallique, le procédé comprenant :
f) une imposition d'un courant électrique et/ou d'une tension électrique entre la première électrode de conductivité et la deuxième électrode de conductivité lorsque la tête de la ou des premières microaiguille(s) est mise en contact avec la peau de l'utilisateur et une première mesure d'une valeur représentative de la conductivité entre la première électrode de conductivité et la deuxième électrode de conductivité,
g) une imposition d'un courant électrique et/ou d'une tension électrique entre la troisième électrode de conductivité et la quatrième électrode de conductivité lorsque le substrat du dispositif est mis en contact avec la peau de l'utilisateur de manière à mettre en contact la première surface de conductivité et la deuxième surface de conductivité avec la peau, et une deuxième mesure d'une valeur représentative de la conductivité entre la troisième électrode de conductivité et la quatrième électrode de conductivité,
h) une étape de détermination de la première conductivité seuil *σₜ₁* à partir de la valeur mesurée lors de la première mesure de l'étape f) et de la deuxième mesure de l'étape g).

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre schématiquement un dispositif conforme à l'invention,
- la figure 2 illustre schématiquement une partie du dispositif conforme à l'invention,
- la figure 3 illustre schématiquement un dispositif comprenant une comprenant une première microaiguille et une deuxième microaiguille,
- la figure 4 illustre schématiquement un dispositif conforme à l'invention comprenant une comprenant une première microaiguille et une surface métallique destinée à être en contact avec la peau de l'utilisateur,
- la figure 5 illustre schématiquement un dispositif comprenant une comprenant une première microaiguille, une deuxième microaiguille, une première surface métallique et une deuxième surface métallique,
- la figure 6 illustre schématiquement un dispositif comprenant une comprenant une première microaiguille, une deuxième microaiguille, une première surface métallique et une deuxième surface métallique,
- la figure 7 illustre schématiquement un système de mesure de la conductivité entre deux électrodes.

Sur l'ensemble des figures, les éléments similaires portent des références identiques.

### DEFINITIONS

On entend par « tête » d'une microaiguille le sommet ou la pointe d'une microaiguille. La tête de la microaiguille désigne dans la présente invention un point. Ainsi, la face métallique des microaiguilles conformes à l'invention recouvre une partie de la microaiguille comprenant sa pointe.

### DESCRIPTION DETAILLEE DE L'INVENTION

### Architecture générale du dispositif

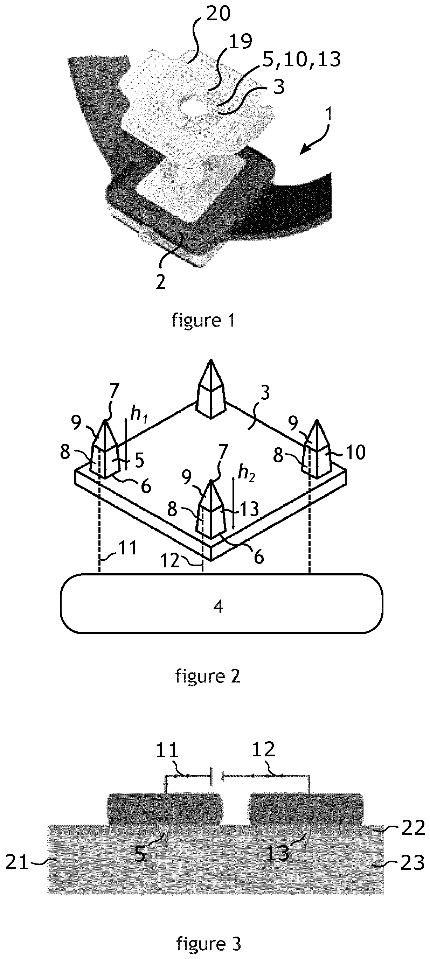

En référence à la figure 1, un dispositif conforme à l'invention comprend un boîtier 2. Le boîtier 2 comprend un substrat 3. Le substrat 3 est propre à venir au contact de la peau d'un utilisateur. A cet effet, le dispositif peut également comprendre un bracelet, monté sur le boîtier 2, permettant de maintenir le substrat 3 au contact de la peau de l'utilisateur. Le boîtier 3 comprend également une unité de contrôle 4. L'unité de contrôle 4 peut par exemple comprendre un microcontrôleur, une mémoire, et un amplificateur de signaux analogiques.

Avantageusement, le boîtier 2 peut comprendre un patch 20 et une capsule 19. Le patch 20 peut comprendre une couche d'adhésif apte à immobiliser le boîtier 2 sur la peau de l'utilisateur. Le patch 20 est propre à recevoir la capsule 19, de sorte à fixer le patch 20 et la capsule 19 de manière amovible. Le patch 20 et la capsule 19 peuvent également être fixés au reste du boîtier 2 de manière amovible.

### Microaiguilles et électrodes

En référence à la figure 2, le dispositif 1 comprend au moins une première microaiguille 5 configurée pour être insérée dans la peau d'un utilisateur. La première microaiguille 5 comprend une base 6 montée fixe sur le substrat 3. En particulier, la première microaiguille 5 et le substrat peuvent être deux pièces fixées l'une à l'autre, mais peuvent aussi être un ensemble monolithique. La première microaiguille 5 comprend une tête 7 ponctuelle, à l'opposé de la base 6. La base 6 et la tête 7 de la première microaiguille 5 sont distantes d'une première hauteur *h₁.* Entre la base 5 exclue et la tête, les parois de la première microaiguille forment une face latérale 8. La face latérale 8 peut par exemple comprendre plusieurs faces planes, ou former un cylindre ou un cône.

La face latérale 8 de la première microaiguille 5 comprend une face métallique 9. La face métallique 9 s'étend de manière continue depuis la tête 7 vers la base 6. Toutefois, la face métallique 9 n'atteint pas la base 6.

La première microaiguille 5 est une microaiguille permettant de mesurer la conductivité du milieu qui l'entoure. Ainsi, la première microaiguille 5 permet de contrôler l'enfoncement d'autres microaiguilles du dispositif 1.

Dans un mode de réalisation de l'invention, le dispositif comprend une pluralité de premières microaiguilles 5.

Le dispositif 1 comprend également une pluralité de microaiguilles de mesure d'analyte 10, configurées pour être insérées dans la peau, et pour détecter la présence d'un ou plusieurs analytes. Une microaiguille de mesure d'analyte 10 comprend typiquement une face métallique, sur laquelle sont fixées des enzymes adaptées à détecter un analyte par conductimétrie. L'analyte peut être choisi parmi du glucose et du lactate. L'enzyme peut être choisi parmi la glucose oxydase et/ou la lactate déshydrogénase. Les microaiguilles de mesure d'analyte 10 sont également montées fixes sur le substrat 3, préférentiellement à moins de 2 cm de la première microaiguille 5.

Le dispositif comprend une première électrode 11 de conductivité. La première électrode 11 de conductivité comprend la face métallique 9 de la ou de chacune des première(s) microaiguille(s) 5. La première électrode 11 de conductivité relie électriquement la face métallique 9 de la ou de chacune des première(s) microaiguille(s) 5 à l'unité de contrôle 4.

En référence aux figures 2,3, 5 et 6, le dispositif comprend préférentiellement au moins une deuxième microaiguille 13 configurée pour être insérée dans la peau d'un utilisateur. La deuxième microaiguille 13 comprend une base 6 montée fixe sur le substrat 3. En particulier, la deuxième microaiguille 13 et le substrat 3 peuvent être deux pièces fixées l'une à l'autre, mais peuvent aussi être un ensemble monolithique. La deuxième microaiguille 13 comprend une tête 7 ponctuelle, à l'opposé de la base 6. La base 6 et la tête 7 de la première microaiguille 5 sont distantes d'une première hauteur *h₂*. Entre la base 5 exclue et la tête 7, les parois de la deuxième microaiguille 13 forment une face latérale 8. La face latérale 8 peut par exemple comprendre plusieurs faces planes, ou former un cylindre ou un cône.

Le dispositif comprend également une deuxième électrode 12 de conductivité. La deuxième électrode 12 de conductivité peut comprendre une surface métallique 24 apte à être mise au contact de la peau de l'utilisateur, ou préférentiellement la deuxième microaiguille 13. La deuxième électrode 12 de conductivité relie électriquement la face métallique 9 de la ou de chacune des deuxième(s) microaiguille(s) 13 à l'unité de contrôle 4, une la surface métallique 24 à l'unité de contrôle 4.

L'unité de contrôle 4 est configurée pour imposer un courant électrique et/ou une tension électrique entre la première électrode 11 de conductivité et la deuxième électrode 12 de conductivité, préférentiellement lorsque le substrat 3 du dispositif est mis en contact avec la peau de l'utilisateur, puis pour mesurer une valeur représentative de la conductivité entre la première électrode 11 de conductivité et la deuxième électrode 12 de conductivité, puis pour enregistrer une donnée représentative d'un enfoncement dans la peau de la ou des microaiguilles de mesure d'analyte suffisant pour une mise en œuvre d'une mesure d'analyte lorsque la valeur représentative de la conductivité est représentative d'une conductivité supérieure à une première conductivité seuil prédéterminée *σₜ₁*.

En effet, les couches de peau en dessous du stratum corneum, en particulier les couches du derme, présentent une conductivité plus élevée que celle du stratum corneum.

Ainsi, en combinant une mesure de conductivité avec l'utilisation de la première microaiguille, il est possible de mesurer la conductivité du milieu au voisinage des microaiguilles de mesure d'analyte, de sorte à contrôler leur enfoncement, de manière transitoire et continue. En particulier, la valeur mesurée quand la première microaiguille 5 n'est pas assez enfoncée est représentative d'une conductivité inférieure à la première conductivité seuil prédéterminée *σₜ₁*. Quand la valeur mesurée est représentative d'une conductivité supérieure à la première conductivité seuil *σₜ₁*, la ou les premières microaiguilles 5 sont enfoncées dans le derme de sorte que les aiguilles de mesure d'analyte sont également suffisamment enfoncées pour mesurer l'analyte. Ainsi, la concentration mesurée par les microaiguilles de mesure d'analyte 10 n'est pas faussée.

Préférentiellement, la surface de la face métallique 9 ou la somme des surfaces des faces métalliques 9 de la ou des premières microaiguilles 5 est supérieure ou égale à 0,35 mm², préférentiellement supérieure à 0,5 mm² et notamment supérieure à 1 mm². En effet, les inventeurs ont découvert que le courant augmente de manière non linéaire à partir d'une surface enfoncée dans le derme supérieure à 0,35 mm², cette augmentation non linéaire permettant de mesurer l'enfoncement des microaiguilles de mesure 10 avec plus de précision.

De plus, les inventeurs ont découvert qu'il était particulièrement avantageux de mesurer la conductivité du tissu dans lequel sont enfoncées les microaiguilles en utilisant au moins une première microaiguille 5 et une deuxième microaiguille 13. En effet, de par la présence dans le derme des deux microaiguilles, la conductivité mesurée est élevée de plus d'un ordre de grandeur au regard d'une conductivité mesurée entre l'extérieur de la peau et une microaiguille, permettant une mesure plus précise de l'enfoncement des microaiguilles du dispositif 1 dans la peau.

Dans un mode de réalisation de l'invention, la première hauteur *h₁* et la deuxième hauteur *h₂* sont strictement différentes. En effet, si les microaiguilles sont accidentellement enlevées du derme, il est possible de mesurer deux diminutions successives de la conductivité mesurée entre la première électrode 11 et la deuxième électrode 12. Ainsi, l'unité de contrôle 4 peut mesurer une première diminution, pour émettre une alerte à l'utilisateur.

Dans un mode de réalisation, la première hauteur *h₁* et la deuxième hauteur *h₂* sont égales, et le substrat 3 présente un enfoncement 14 sur lequel est monté fixe la base 6 de la première microaiguille 5 ou la base 6 de la deuxième microaiguille 13. Ainsi, il est possible de produire la première microaiguille 5 et la deuxième microaiguille 13 en utilisant les mêmes procédés de fabrication, aboutissant à la même hauteur de microaiguille, tout en fabricant un dispositif 1 dans lequel la tête 7 de la première microaiguille 5 et la tête 7 de la deuxième microaiguille 13 sont à des distances différentes d'un plan passant par le substrat 3. Ainsi, il est possible de mesurer deux diminutions successives de la conductivité mesurée entre la première électrode 11 et la deuxième électrode 12 lors du départ des microaiguilles de la peau. L'unité de contrôle 4 peut mesurer une première diminution, et émettre une alerte à l'utilisateur. Avantageusement, le dispositif 1 comprend une pluralité de premières microaiguilles 5, les hauteurs *h₁* d'au moins deux premières microaiguilles 5 étant strictement différentes entre-elles et/ou les hauteurs *h₁* des premières microaiguilles 5 étant égales entre-elles, le substrat 3 présentant un enfoncement 14 sur lequel est monté fixe la base 6 d'au moins une des premières microaiguilles 5. Ainsi, il est possible de mesurer des variations de conductivités lors de l'enfoncement ou lors du départ des premières microaiguilles de la peau, tout en laissant la deuxième microaiguille 13 enfoncée dans le derme.

Avantageusement, la face métallique 9 de la première microaiguille 5 et la face métallique 9 de la deuxième microaiguille 13 s'étendent depuis chacune des têtes de chaque microaiguille jusqu'à des hauteurs strictement différentes. Ainsi, il est possible de mesurer des variations de conductivité lors de l'enfoncement ou lors du départ des premières microaiguilles 5 de la peau, tout en munissant le dispositif de microaiguilles de la même hauteur, ce qui simplifie leur fabrication.

Avantageusement, la face métallique 9 de la première microaiguille 5 s'étend depuis la tête 7 de la première microaiguille 5 jusqu'à une partie de la face latérale 8 distante de plus de *h*/10, préférentiellement de plus de h/5, et notamment de plus de h/3 du substrat 3. La face métallique 9 de la première microaiguille 5 peut s'étendre depuis la tête 7 de chaque microaiguille jusqu'à une partie de la face latérale 8 distante de moins de 9.h/10, préférentiellement de moins de 4.h/5, et notamment de moins de 2.h/3 du substrat. Ainsi, il est possible de maximiser la surface de la face métallique sur chacune des microaiguilles, tout en évitant de mesurer une composante de la conductivité imputable à la sueur sur la peau. En effet, quand une première microaiguille 5 et une deuxième microaiguille 13 sont enfoncées dans la peau, la base de chaque microaiguille peut être en contact avec la sueur de la peau.

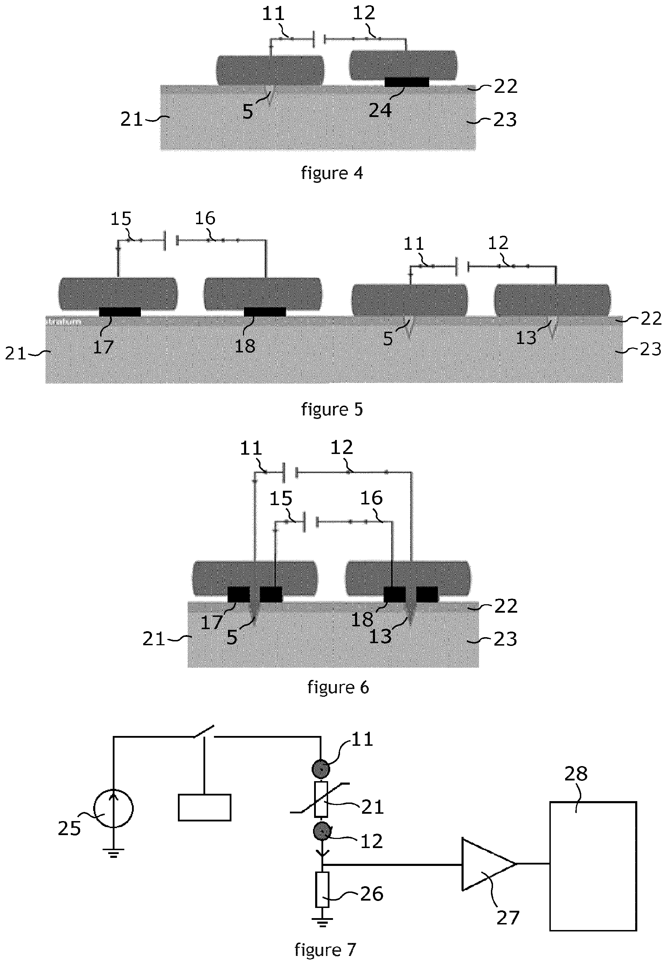

En référence à la figure 4, le dispositif peut comprendre une troisième électrode de conductivité 15, et préférentiellement une quatrième électrode de conductivité 16. Le substrat 3 peut comprendre une première surface 17 de conductivité métallique propre à venir au contact de la peau, et préférentiellement une deuxième surface 18 de conductivité métallique propre à venir au contact de la peau. La troisième électrode 15 de conductivité relie électriquement l'unité de contrôle 4 à la première surface 17 de conductivité métallique, et préférentiellement la quatrième électrode 16 de conductivité relie électriquement l'unité de contrôle 4 à la deuxième surface 18 de conductivité métallique. Ainsi, il est possible de déterminer à l'aide d'une mesure d'impédance mise en œuvre par la première surface 17 et par la deuxième surface 18 une valeur de la première conductivité seuil prédéterminée *σₜ₁* ajustée à la peau de l'utilisateur de manière personnalisée. En effet, l'épaisseur de peau peut varier d'un individu à l'autre.

Avantageusement, la valeur de la première conductivité seuil *σₜ₁* est supérieure ou égale à 0,05 S/m, notamment supérieure à 0,1 S/m et préférentiellement supérieure à 0,3 S/m. Ainsi, il est possible de distinguer le derme du stratum corneum par la mesure d'une valeur représentative de la conductivité.

Avantageusement, quand un courant est imposé par l'unité de contrôle 4 pour mesurer une valeur représentative de la conductivité entre la première électrode 11 de conductivité et la deuxième électrode 12 de conductivité, le courant est compris entre 1 nA et 100 µA.

En référence à la figure 6, les électrodes de conductivité peuvent être imbriquées aux surfaces métalliques : avantageusement, la première surface 17 métallique ou la deuxième surface 18 peut être agencée autour de la base 6 de la première microaiguille 5 ou de la deuxième microaiguille 13. Ainsi, il est possible réduire la superficie utilisée sur le substrat par les différentes électrodes.

Avantageusement, une tension et/ou un courant continu(s) ou alternatif(s) peut être imposé pour mesurer la valeur représentative de la conductivité entre la première électrode 11 et la deuxième électrode 12.

### Système de mesure

En référence à la figure 7, le dispositif 1 comprend une source de courant et/ou de tension 25, adaptée(s) pour imposer une tension et/ou un courant entre la première électrode 11 de conductivité et la deuxième électrode 12 de conductivité. Avantageusement, le système de mesure comprend un pont diviseur de tension. Une tension peut être mesurée aux bornes d'une résistance de référence 26, agencée en série avec l'impédance, par exemple de la peau 21, formée entre la première électrode 11 et la deuxième électrode 12. Ainsi, on peut imposer un courant et une tension à la première électrode 11, et mesurer une tension représentative de la conductivité entre la première électrode 11 et la deuxième électrode 12 aux bornes de la résistance de référence 26. Cette tension peut être amplifiée par un amplificateur de tension 27, puis mesurée par un microcontrôleur 28.

### Configuration de l'unité de contrôle et procédé(s) de mesure de l'enfoncement d'une microaiguille

Le dispositif peut être configuré pour éviter qu'une sueur trop importante sur la peau ne perturbe la mesure de l'enfoncement de la première microaiguille 5. L'unité de contrôle 4 peut être configurée pour enregistrer une donnée représentative d'une humidité intense sur la peau de l'utilisateur lorsque la valeur mesurée lors de l'étape a) d'imposition d'un courant électrique et/ou d'une tension électrique est représentative d'une conductivité supérieure à une deuxième conductivité seuil *σₜ₂*, la valeur de la deuxième conductivité seuil *σₜ₂* étant préférentiellement supérieure à 1 S/m, notamment supérieure à 0,7 S/m. En effet, la conductivité du derme est limitée. La mesure d'une conductivité supérieure à la valeur maximale de la conductivité du derme indique que la conductivité mesurée est celle de la sueur à la surface de la peau.

L'unité de contrôle 4 peut être avantageusement configurée pour enregistrer une donnée représentative d'un enfoncement insuffisant d'une première microaiguille 5 dans la peau de l'utilisateur lorsque la valeur représentative de la conductivité est représentative d'une conductivité inférieure à la première conductivité seuil *σₜ₁* prédéterminée. Ainsi, l'utilisateur peut être informé de la nécessité de replacer le dispositif 1 de manière à enfoncer suffisamment les microaiguilles. Il peut être également prévenu d'un enfoncement insuffisant des microaiguilles avant que les microaiguilles de mesure d'analyte ne soient sorties de la peau.

L'unité de contrôle 4 peut avantageusement être configurée pour :
- mesurer la concentration C₀ d'un analyte présent dans un liquide interstitiel,
- enregistrer une donnée représentative une donnée représentative d'une humidité modérée sur la peau de l'utilisateur et d'un enfoncement d'une première microaiguille 5 insuffisant de la peau de l'utilisateur lorsque la conductivité représentée par la valeur mesurée est supérieure à la valeur de la première conductivité électrique seuil *σₜ₁*, et lorsque la concentration de l'analyte de mesure est inférieure à une première concentration C₁ prédéterminée ou supérieure à une deuxième concentration C₂ prédéterminée, la première concentration étant préférentiellement inférieure à 2 mM inclus, et notamment à 0,5 mM inclus, la deuxième concentration étant préférentiellement supérieure à 20 mM inclus. En effet, la conductivité de la sueur à la surface de la peau peut être égale à celle du derme. Pour différencier ces mesures, on peut avantageusement discriminer le milieu mesuré par la mesure de la concentration d'analyte. Avantageusement, la sueur en surface de la peau présente une concentration en glucose plus basse que dans le derme. Ainsi, si la concentration en glucose est inférieure à 2 mM, notamment à 0,5 mM, on peut conclure que la mesure de conductivité est mise en œuvre sur la surface de la peau. Avantageusement, la sueur en surface de la peau présente une concentration en lactate plus élevée que dans le derme. Ainsi, si la concentration en lactate est supérieure à 20 mM, on peut conclure que la mesure de conductivité est mise en œuvre sur la surface de la peau.

L'unité de contrôle 4 est avantageusement configurée pour que l'imposition d'un courant électrique et/ou d'une tension électrique entre la première électrode 11 de conductivité et la deuxième électrode 12 de conductivité soit mise en œuvre pendant moins de 300 ms, notamment pendant moins de 200 ms, et préférentiellement pendant moins de 100 ms. Ainsi, le derme n'est pas endommagé pendant la mesure. En effet, les inventeurs ont mesuré qu'une brûlure visible du derme apparaissait pour des durées de mesure plus longues.

Avantageusement, un procédé conforme à l'invention peut permettre de calibrer le dispositif, et en particulier de calculer la première conductivité seuil prédéterminée *σₜ₁*. Un procédé de calibration peut comprendre une imposition d'un courant électrique et/ou d'une tension électrique entre la première électrode 11 de conductivité et la deuxième électrode 12 de conductivité lorsque la tête 7 de la ou des premières microaiguille(s) est mise en contact avec la peau de l'utilisateur et une première mesure d'une valeur représentative de la conductivité entre la première électrode 11 de conductivité et la deuxième électrode 12 de conductivité, puis une imposition d'un courant électrique et/ou d'une tension électrique entre la première électrode 11 de conductivité et la deuxième électrode 12 de conductivité de conductivité lorsque le substrat 3 du dispositif est mis en contact avec la peau de l'utilisateur de manière à enfoncer la ou les premières microaiguilles 5 dans la peau, et une deuxième mesure d'une valeur représentative de la conductivité entre la première électrode 11 de conductivité et la deuxième électrode 12 de conductivité, et enfin une étape de détermination de la première conductivité seuil *σₜ₁* à partir de la première mesure et à partir de la deuxième mesure.

Ainsi, il est possible de prendre en compte dans la détermination de la première conductivité seuil *σₜ₁* la conductivité de la surface de la peau d'un utilisateur.

Le procédé de calibration peut également comprendre une imposition d'un courant électrique et/ou d'une tension électrique entre la première électrode 11 de conductivité et la deuxième électrode 12 de conductivité lorsque la tête 7 de la ou des premières microaiguille(s) 5 est mise en contact avec la peau de l'utilisateur et une première mesure d'une valeur représentative de la conductivité entre la première électrode 11 de conductivité et la deuxième électrode 12 de conductivité, puis une imposition d'un courant électrique et/ou d'une tension électrique entre la troisième électrode 15 de conductivité et la quatrième électrode 16 de conductivité lorsque le substrat 3 du dispositif est mis en contact avec la peau de l'utilisateur de manière à mettre en contact la première surface 17 de conductivité et la deuxième surface 18 de conductivité avec la peau, et une deuxième mesure d'une valeur représentative de la conductivité entre la troisième électrode 15 de conductivité et la quatrième électrode 16 de conductivité, et une étape de détermination de la première conductivité seuil *σₜ₁* à partir de la valeur mesurée lors de la première mesure et de la deuxième mesure.

## Revendications

1. Dispositif (1) de mesure d'analyte présent dans un liquide interstitiel, le dispositif (1) comprenant :
- un boîtier (2) comprenant un substrat (3),
- une unité de contrôle (4), le boîtier (2) comprenant l'unité de contrôle (4),
- au moins une première microaiguille (5) configurée pour être insérée dans la peau d'un utilisateur,
la première microaiguille (5) comprenant une base (6) montée fixe sur le substrat (3) et une tête (7) ponctuelle de la première microaiguille (5), la base (6) et la tête (7) de la première microaiguille (5) étant distantes d'une première hauteur *h₁*, la première microaiguille (5) présentant une face latérale (8) comprise entre la base exclue et la tête (7), la face latérale (8) comprenant une face métallique (9) s'étendant continûment depuis la tête (7),
- une pluralité de microaiguilles de mesure d'analyte (10) configurées pour être insérées dans la peau,
- une première électrode (11) de conductivité et une deuxième électrode (12) de conductivité, la première électrode (11) de conductivité comprenant la face métallique (9) de la ou de chacune des première(s) microaiguille(s) (5), et reliant électriquement la face métallique (9) de la ou de chacune des première(s) microaiguille(s) (5) à l'unité de contrôle (4),
**caractérisé en ce que** l'unité de contrôle (4) est configurée pour :
a) imposer un courant électrique et/ou une tension électrique entre la première électrode (11) de conductivité et la deuxième électrode (12) de conductivité lorsque le substrat (3) du dispositif est mis en contact avec la peau de l'utilisateur, et mesurer une valeur représentative de la conductivité entre la première électrode (11) de conductivité et la deuxième électrode (12) de conductivité,
b) enregistrer une donnée représentative d'un enfoncement dans la peau de la ou des microaiguilles de mesure d'analyte suffisant pour une mise en œuvre d'une mesure d'analyte lorsque la valeur mesurée lors de l'étape a) est représentative d'une conductivité supérieure à une première conductivité seuil prédéterminée *σₜ₁*.

2. Dispositif selon la revendication 1, dans laquelle la surface de la face métallique (9) ou la somme des surfaces des faces métalliques (9) est supérieure ou égale à 0,35 mm², préférentiellement supérieure à 0,5 mm² et notamment supérieure à 1 mm².

3. Dispositif selon la revendication 1, comprenant une deuxième microaiguille (13), la deuxième microaiguille (13) comprenant une base (6) de la deuxième microaiguille montée fixe sur le substrat (3) et une tête (7) ponctuelle de la deuxième microaiguille (13), la base (6) et la tête (7) de la deuxième microaiguille (13) étant distantes d'une deuxième hauteur *h₂*, la deuxième microaiguille (13) présentant une face latérale (8) comprise entre la base (6) de la deuxième microaiguille (13) exclue et la tête (7) de la deuxième microaiguille (13), la face latérale (8) de la deuxième microaiguille (13) comprenant une face métallique (9) s'étendant continûment depuis la tête (7), la deuxième électrode (12) de conductivité comprenant la face métallique (9) de la deuxième microaiguille (13) et reliant électriquement la face métallique (9) de la deuxième microaiguille (13) à l'unité de contrôle (4).

4. Dispositif selon la revendication 3, dans lequel la première hauteur *h₁* et la deuxième hauteur *h₂* sont strictement différentes.

5. Dispositif selon la revendication 3, dans lequel la première hauteur *h₁* et la deuxième hauteur *h₂* sont égales, et dans lequel le substrat (3) présente un enfoncement (14) sur lequel est monté fixe la base (6) de la première microaiguille (5) ou la base (6) de la deuxième microaiguille (13).

6. Dispositif selon l'une des revendications 1 à 5, comprenant une pluralité de premières microaiguilles (5),
les hauteurs *h₁* d'au moins deux premières microaiguilles (5) étant strictement différentes entre-elles et/ou
les hauteurs *h₁* des premières microaiguilles (5) étant égales entre-elles, le substrat (3) présentant un enfoncement (14) sur lequel est monté fixe la base (6) d'au moins une des premières microaiguilles (5).

7. Dispositif selon l'une des revendications 1 à 6, dans lequel la face métallique (9) de la première microaiguille (5) s'étend depuis la tête (7) de la première microaiguille (5) jusqu'à une partie de la face latérale (8) distante de plus de *h*/10, préférentiellement de plus de h/5, et notamment de plus de h/3 du substrat (3).

8. Dispositif selon l'une des revendications 1 à 7, dans lequel la face métallique (9) de la première microaiguille (5) s'étend depuis la tête (7) de chaque microaiguille jusqu'à une partie de la face latérale (8) distante de moins de 9.h/10, préférentiellement de moins de 4.h/5, et notamment de moins de 2.h/3 du substrat.

9. Dispositif selon l'une des revendications 2 à 8, dans lequel la face métallique (9) de la première microaiguille (5) et la face métallique (9) de la deuxième microaiguille (13) s'étendent depuis chacune des têtes de chaque microaiguille jusqu'à des hauteurs strictement différentes.

10. Dispositif selon l'une des revendications 1 à 9, comprenant une troisième électrode de conductivité (15), et préférentiellement une quatrième électrode de conductivité (16), le substrat (3) comprenant une première surface (17) de conductivité métallique, et préférentiellement une deuxième surface (18) de conductivité métallique, la troisième électrode (15) de conductivité reliant électriquement l'unité de contrôle (4) à la première surface (17) de conductivité métallique, et préférentiellement la quatrième **électrode** (16) de conductivité reliant électriquement l'unité de contrôle (4) à la deuxième surface (18) de conductivité métallique.

11. Dispositif selon l'une des revendications 1 à 10, dans lequel la valeur de la première conductivité seuil *σₜ₁* est supérieure ou égale à 0,05 S/m, notamment supérieure à 0,1 S/m et préférentiellement supérieure à 0,3 S/m.

12. Dispositif selon l'une des revendications 1 à 11, dans lequel l'unité de contrôle (4) est configurée pour enregistrer une donnée représentative d'une humidité intense sur la peau de l'utilisateur lorsque la valeur mesurée lors de l'étape a) est représentative d'une conductivité supérieure à une deuxième conductivité seuil *σₜ₂,* la valeur de la deuxième conductivité seuil *σₜ₂* étant préférentiellement supérieure à 0,7 S/m.

13. Dispositif selon l'une des revendications 1 à 12, dans lequel l'unité de contrôle (4) est configurée pour enregistrer une donnée représentative d'un enfoncement d'une première microaiguille (5) insuffisant de la peau de l'utilisateur lorsque la valeur mesurée lors de l'étape a) est représentative d'une conductivité inférieure à la première conductivité seuil *σₜ₁* prédéterminée.

14. Dispositif selon l'une des revendications 1 à 13, dans lequel l'unité de contrôle (4) est configurée pour :
- mesurer la concentration C₀ d'un analyte présent dans un liquide interstitiel,
- enregistrer une donnée représentative une donnée représentative d'une humidité modérée sur la peau de l'utilisateur et d'un enfoncement d'une première microaiguille (5) insuffisant de la peau de l'utilisateur lorsque la valeur de la conductivité mesurée lors de l'étape a) est supérieure à la valeur de la première conductivité électrique seuil *σₜ₁*, et lorsque la concentration de l'analyte de mesure est inférieure à une première concentration C₁ prédéterminée ou supérieure à une deuxième concentration C₂ prédéterminée, la première concentration étant préférentiellement inférieure à 2 mM inclus, et notamment à 0,5 mM inclus, la deuxième concentration étant préférentiellement supérieure à 20 mM inclus.

15. Procédé de détection de l'enfoncement d'une microaiguille de mesure d'analyte (10) d'un dispositif (1) conforme à la revendication 1 au travers de la peau, comprenant au moins :
a) une imposition d'un courant électrique et/ou d'une tension électrique entre la première électrode (11) de conductivité et la deuxième électrode (12) de conductivité lorsque le substrat (3) du dispositif est mis en contact avec la peau de l'utilisateur et de mesure d'une valeur représentative de la conductivité entre la première électrode (11) de conductivité et la deuxième électrode (12) de conductivité,
b) l'enregistrement d'une donnée représentative d'un enfoncement dans la peau de la ou des microaiguilles de mesure d'analyte suffisant pour une mise en œuvre d'une mesure d'analyte lorsque la valeur mesurée lors de l'étape a) est représentative d'une conductivité supérieure à une première conductivité seuil prédéterminée *σₜ₁*.

16. Procédé selon la revendication 15, dans lequel, lors de l'étape a), l'imposition d'un courant électrique et/ou d'une tension électrique entre la première électrode (11) de conductivité et la deuxième électrode (12) de conductivité est mise en œuvre pendant moins de 300 ms, notamment pendant moins de 200 ms, et préférentiellement pendant moins de 100 ms.

17. Procédé de calibration d'un dispositif conforme à la revendication 1, comprenant :
c) une imposition d'un courant électrique et/ou d'une tension électrique entre la première électrode (11) de conductivité et la deuxième électrode (12) de conductivité lorsque la tête (7) de la ou des premières microaiguille(s) est mise en contact avec la peau de l'utilisateur et une première mesure d'une valeur représentative de la conductivité entre la première électrode (11) de conductivité et la deuxième électrode (12) de conductivité,
d) une imposition d'un courant électrique et/ou d'une tension électrique entre la première électrode (11) de conductivité et la deuxième électrode (12) de conductivité de conductivité lorsque le substrat (3) du dispositif est mis en contact avec la peau de l'utilisateur de manière à enfoncer la ou les premières microaiguilles (5) dans la peau, et mesure d'une valeur représentative de la conductivité entre la première électrode (11) de conductivité et la deuxième électrode (12) de conductivité,
e) une étape de détermination de la première conductivité seuil *σₜ₁* à partir de la valeur mesurée lors de l'étape c) et à partir de la valeur mesurée lors de l'étape d).

18. Procédé de calibration d'un dispositif conforme à la revendication 1, le dispositif comprenant en outre une troisième électrode de conductivité (15) et une quatrième électrode de conductivité (16), le substrat (3) comprenant une première surface (17) de conductivité métallique, et une deuxième surface (18) de conductivité métallique, la troisième électrode (15) de conductivité reliant électriquement l'unité de contrôle (4) à la première surface (17) de conductivité métallique, et la quatrième électrode (16) de conductivité reliant électriquement l'unité de contrôle (4) à la deuxième surface (18) de conductivité métallique, le procédé comprenant :
f) une imposition d'un courant électrique et/ou d'une tension électrique entre la première électrode (11) de conductivité et la deuxième électrode (12) de conductivité lorsque la tête (7) de la ou des premières microaiguille(s) (5) est mise en contact avec la peau de l'utilisateur et une première mesure d'une valeur représentative de la conductivité entre la première électrode (11) de conductivité et la deuxième électrode (12) de conductivité,
g) une imposition d'un courant électrique et/ou d'une tension électrique entre la troisième électrode (15) de conductivité et la quatrième électrode (16) de conductivité lorsque le substrat (3) du dispositif est mis en contact avec la peau de l'utilisateur de manière à mettre en contact la première surface (17) de conductivité et la deuxième surface (18) de conductivité avec la peau, et une deuxième mesure d'une valeur représentative de la conductivité entre la troisième électrode (15) de conductivité et la quatrième électrode (16) de conductivité,
h) une étape de détermination de la première conductivité seuil *σₜ₁* à partir de la valeur mesurée lors de la première mesure de l'étape f) et de la deuxième mesure de l'étape g).
